# EUROPÄISCHE PATENTSCHRIFT

(11) **EP 1 968 647 B1**
(45) Veröffentlichungstag und Bekanntmachung des Hinweises auf die Patenterteilung: **16.10.2013**
(21) Anmeldenummer: 06840896.2
(22) Anmeldetag: 29.12.2006
(51) Int. Cl.: A61K 49/00

(54) **DIAGNOSTIKUM UND VERFAHREN ZUR UNTERSUCHUNG VON STOFFWECHSELVORGÄNGEN IM GEHIRN**
DIAGNOSTIC SUBSTANCE AND METHOD FOR THE ANALYSIS OF METABOLISM PROCESSES IN THE BRAIN
AGENT DE DIAGNOSTIC ET PROCEDE D'EXAMEN DE PROCESSUS DU METABOLISME DANS LE CERVEAU

(30) Priorität: 30.12.2005 DE 102005063174
(43) Veröffentlichungstag der Anmeldung: 17.09.2008
(73) Patentinhaber: Leibniz-Institut für Neurobiologie, 39118 Magdeburg (DE)
(72) Erfinder: ZIABREVA, Iryna, Wardley Gateshead NE108WJ (GB); HENRICH-NOACK, Petra, 39120 Magdeburg (DE); GOLDSCHMIDT, Jürgen, 39104 Magdeburg (DE); BALDAUF, Kathrin, 39291 Möser (DE); SCHEICH, Henning, 39326 Samswegen (DE); REYMANN, Klaus G., 39167 Niederndodeleben (DE); SCHRÖDER, Ulrich H., 97776 Eussenheim (DE); PFORTE, Claudia, 06179 Schochwitz (DE); RIEK-BURCHARDT, Monika, 39638 Kloster Neuendorf (DE)
(74) Vertreter: Schubert, Klemens
(86) Internationale Anmeldenummer: PCT/DE2006/002345
(87) Internationale Veröffentlichungsnummer: WO 2007/076848

(56) Entgegenhaltungen:
- VAN ROYEN E A ET AL: "CEREBRAL BLOOD FLOW IMAGING WITH THALLIUM-201 DIETHYLDITHIOCARBAMATE SPECT" JOURNAL OF NUCLEAR MEDICINE, Bd. 28, Nr. 2, 1987, Seiten 178-183, XP002482329 ISSN: 0161-5505
- DE BRUINE J F ET AL: "THALLIUM-201 DIETHYLDITHIOCARBAMATE AS AN ALTERNATIVE FOR IODINE-123 AMPHETAMINE IN BRAIN IMAGING WITH SPECT" BIERSACK, H. J. AND C. WINKLER (ED.). AMPHETAMINES AND PH-SHIFT AGENTS FOR BRAIN IMAGING: BASIC RESEARCH AND CLINICAL RESULTS. VIII+186P. WALTER DE GRUYTER: BERLIN, WEST GERMANY; NEW YORK, NEW YORK, USA. ILLUS, 1986, Seiten 51-58, XP002482330 ISSN: 3-11-010772-4 0-89925-156-0
- LEAR J L ET AL: "Autoradiographic comparison of thallium-201 diethyldithiocarbamate, isopropyliodoamphetamine and iodoantipyrine as cerebral blood flow tracers." JOURNAL OF NUCLEAR MEDICINE : OFFICIAL PUBLICATION, SOCIETY OF NUCLEAR MEDICINE APR 1987, Bd. 28, Nr. 4, April 1987 (1987-04), Seiten 481-486, XP002482331 ISSN: 0161-5505
- BALLINGER JAMES R ET AL: "TECHNETIUM-99m DIETHYLDITHIOCARBAMATE (DDC): COMPARISON WITH THALLIUM-201 DDC AS AN AGENT FOR BRAIN IMAGING" APPL RADIAT ISOT 1987, Bd. 38, Nr. 8, 1987, Seiten 665-668, XP002482332 in der Anmeldung erwähnt
- LIMBURG M ET AL: "Single-photon emission computed tomography and early death in acute ischemic stroke" STROKE 1990 US, Bd. 21, Nr. 8, 1990, Seiten 1150-1155, XP002482333 ISSN: 0039-2499
- SCOPINARO F ET AL: "ENCEPHALIC UPTAKE OF THALLIUM-201-LABELED DIETHYLDITHIOCARBAMATE DDC" EUROPEAN JOURNAL OF NUCLEAR MEDICINE, Bd. 15, Nr. 8, 1989, Seite 575, XP002482334 & EUROPEAN ASSOCIATION OF NUCLEAR MEDICINE CONGRESS, STRASBOURG, FRANCE, AUGUST 28-SEPTEMBER 1, 1989. ISSN: 0340-6997
- VERHOEFF N P L G ET AL: "A COMPARISON OF THALLIUM-201-LABELED DDC AND TECHNETIUM-99M-LABELED HMPAO SPECT AND CT IN PATIENTS WITH A CEREBROVASCULAR ACCIDENT" EUROPEAN JOURNAL OF NUCLEAR MEDICINE, Bd. 16, Nr. 7, 1990, Seite 559, XP002482335 & MEETING OF THE EUROPEAN ASSOCIATION OF NUCLEAR MEDICINE CONGRESS, AMSTERDAM, THE NETHERLANDS, MAY 20 ISSN: 0340-6997
- LIMBURG M ET AL: "rCBF-SPECT in brain infarction: when does it predict outcome?" JOURNAL OF NUCLEAR MEDICINE : OFFICIAL PUBLICATION, SOCIETY OF NUCLEAR MEDICINE MAR 1991, Bd. 32, Nr. 3, März 1991 (1991-03), Seiten 382-387, XP002482336 ISSN: 0161-5505
- FRITS DE BRUINE J ET AL: "SPET BRAIN IMAGING WITH THALLIUM-201 DIETHYLDITHIOCARBAMATE IN ACUTE ISCHEMIC STROKE" EUROPEAN JOURNAL OF NUCLEAR MEDICINE, Bd. 17, Nr. 5, 1990, Seiten 248-251, XP002482337 ISSN: 0340-6997
- OVERBY C ET AL: "EFFECT OF BLOOD-BRAIN BARRIER DISRUPTION ON THALLIUM-201 DIETHYLDITHIOCARBAMATE DISTRIBUTION IN RAT BRAINS" JOURNAL OF NUCLEAR MEDICINE, Bd. 27, Nr. 5, 1986, Seite 736, XP002482338 & SYMPOSIUM ON METABOLIC IMAGING WITH NUCLEAR MAGNETIC RESONANCE, SINGLE PHOTON EMISSION COMPUTED TOMO ISSN: 0161-5505
- GOLDSCHMIDT J ET AL: "High-resolution mapping of neuronal activity by thallium autometallography" NEUROIMAGE, ACADEMIC PRESS, ORLANDO, FL, US, Bd. 23, Nr. 2, 1. Oktober 2004 (2004-10-01), Seiten 638-647, XP004616743 ISSN: 1053-8119 in der Anmeldung erwähnt
- BLAU M: "Radiotracers for functional brain imaging." SEMINARS IN NUCLEAR MEDICINE OCT 1985, Bd. 15, Nr. 4, Oktober 1985 (1985-10), Seiten 329-334, XP002482339 ISSN: 0001-2998

## Beschreibung

Die vorliegende Erfindung betrifft die Verwendung eines Diagnostikums, das mindestens einen Komplex aus lipophilen Anionen und Metallionen enthält, zur Untersuchung von Stoffwechselvorgängen im Gehirn und/oder zentralen Nervensystem (ZNS).

Veränderungen der Aktivität und des Stoffwechsels von Neuronen und Gliazellen sind von Veränderungen in der Aufnahmerate und den intra- und extrazellulären Konzentrationen zahlreicher Kationen (z. B. Na+, K+, Ca++, Mg++, Zn++) begleitet. Bei pathologischen Prozessen, insbesondere Ischämien, Tumoren, Entzündungen und neurodegenerativen Erkrankungen (Demenzen, Alzheimer-Erkrankung) kommt es zu veränderter neuronaler und Gliazellaktivität und zu Verschiebungen in den Kationengleichgewichten. Überdies können insbesondere bei degenerativen Veränderungen Gewebsbestandteile ein verändertes Kationenbindungsverhalten aufweisen.

Bisher können Veränderungen im Kationen-Metabolismus im ZNS in der Routine-Diagnostik noch nicht untersucht werden.

Bekannt sind Versuche zur Untersuchung des Kationen-Metabolismus mittels Kernresonanz-Spektroskopie, wobei eine Voraussetzung ist, dass messbare Isotope vorliegen. Diese Bedingung ist zwar im Fall der Messung von Kaliumionen erfüllt, jedoch ist die bei diesen Untersuchungen erreichte räumliche Auflösung in der Regel schlechter als bei Isotopen-Untersuchungen mittels Gamma-Kamera. Und im Fall anderer Isotope, wie zum Beispiel des Calciums, ist die Untersuchung mittels NMR-Spektroskopie überhaupt nicht möglich.

Lediglich im Tierversuch kann - nach Öffnung der Blut-Hirnschranke - das paramagnetische Mangan (Mn++) als Tracer für den Calcium-Stoffwechsel eingesetzt werden. Eine Übertragung dieses Verfahrens auf den Menschen ist bisher jedoch nicht möglich, da es noch nicht gelungen ist, nicht-toxische Mengen von Mangan durch die Blut-Hirn-schranke zu schleusen und für die Magnetresonanz-Bildgebung beim Menschen einzusetzen.

Das einzige bislang einsetzbare Verfahren zur direkten Messung metabolischer Veränderungen im ZNS ist die Positronen-Emissions-tomographische Messung des Glukose-Metabolismus (¹⁸Fluor-Deoxyglucose-PET).

Aufgrund des großen apparativen Aufwandes wird dieses Verfahren jedoch nur an wenigen ausgewählten Kliniken genutzt und ist schwerlich als Diagnoseverfahren auf den Bereich von Arztpraxen übertragbar.

In der Regel werden heute die Veränderungen im Ionen-Metabolismus indirekt, zum Beispiel über Veränderungen in der Beweglichkeit von Wassermolekülen in der Magnetresonanztomographie (MRT) oder über zerebrale Blutflussmessungen untersucht.

Eingesetzt werden bei diesen so genannten Tracer-Techniken unter anderem Komplexbildner, welche die Isotope bestimmter Schwermetallionen komplexieren. Das in diesen Komplexen eingebundene Schwermetallion spielt lediglich die Rolle eines "Reporters", der anzeigen soll, wohin die Komplexverbindung im Körper diffundiert. Eigentliches Diagnostikum ist die komplexierende Verbindung.

Nachteilig bei diesen indirekten Messung ist jedoch, dass ausschließlich die Folgeveränderungen des gestörten Metabolismus - wie veränderter Blutfluss oder ein mittels Magnetresonanz messbares verändertes Resonanzverhalten von Wasserprotonen - gemessen wird. Und es ist bis heute nur ansatzweise geklärt, wie und unter welchen Bedingungen Störungen des zellulären Metabolismus zu Blutflussränderungen oder Veränderungen der Wasserprotonresonanzen führen.

Eine direkte Messung des Kationen-Metabolismus hat hingegen Vorteil, dass damit ein direkter Einblick in den zellulären Metabolismus geboten wird. Bei der Therapiekontrolle könnte die direkte Bestimmung des Kationen-Metabolismus noch bedeutsamer sein, als für die reine Diagnosestellung einer ZNS-Erkrankung, da bislang unklar ist, wie sich die Erholung des Metabolismus auf die oben genannten Folgeveränderungen (Blutfluss, Wasserprotonresonanz) auswirkt. Aus diesem Grund ist es durchaus denkbar, dass der zelluläre Metabolismus sich erholt, ohne dass die mit Magnetresonanz messbaren indirekten Wassersignale sich verändern oder noch bevor diese sich verändern.

Bei den oben erwähnten indirekten Verfahren zur Untersuchung des Ionenmetabolismus ist die Selektion eines geeigneten Tracers von allergrößter Bedeutung.

Dabei ist eines der wichtigsten Kriterien bei der Auswahl des Komplexbildners und des zu komplexierenden Metallions in diesen Verfahren, die Stabilität der Komplexverbindung in physiologischer Umgebung. Denn nur ein intakter Tracer, in dem das Metallion noch an das Komplexierungsmittel gebunden ist, ermöglicht den oben beschrieben Nachweis des Komplexierungsmittels mit Verfahren, bei denen Metallionen detektiert werden.

Bekannt ist in diesem Zusammenhang beispielsweise die Verwendung der nichtradioaktiven Isotope des Thalliums und des Gammastrahlers ²⁰¹Tl, die Verwendung nichtradioaktiver Isotope des Kobalts, des Gammastrahlers ⁵⁷Co und des Positronenemitters ⁵⁵Co, der nichtradioaktiven Isotope des Mangans und der Positronenemitter ^{52m}Mn, sowie der nichtradioaktiven Isotope von Blei, Eisen und Nickel.

Der Nachweis des Tracers, und genauer gesagt des Reportes im Tracer, erfolgt dann bei paramagnetischen Isotopen (Mangan, Kobalt, Eisen) mithilfe kernmagnetischer Resonanzverfahren, Positronenemissionstomographie, PET, für den Nachweis der Positronenemitter und Single-Photon-Emmissionstomographie, SPET, für den Nachweis der Gammastrahler.

Diese Stabilität der Tracer-Moleküle, die bewirkt, dass nahezu keine freien Metallionen im untersuchten Gewebe zurückgehalten werden, erfordert jedoch gerade bei bildgebenden Nachweismethoden, dass diese ausreichend schnell sind, um ein Bild in dem Zeitraum zu erzeugen, in welchem sich der Tracer im Untersuchungsbereich befindet. In einer Arbeit von Ballinger et al., Appl. Radiat. Isotop. Vol. 38, Nr. 8, Seiten 665-668, 1987, wurde genau dieses Problem im Zusammenhang mit SPECT-Untersuchungen mit einer Gamma-Kamera bei der Messung des zerebralen Blutflusses diskutiert. Da die Gamma-Kamera zum damaligen Zeitpunkt 20 bis 40 Minuten benötigte, um ein Bild zu akkumulieren, wurde die Eignung zweier Tracer-Moleküle für dieses Verfahren diskutiert. Es wurde ein Vergleich vorgenommen zwischen zwei lipophilen Komplexen, nämlich Technetium-99m-Diethydithiocarbamat (^{99m}Tc-DDC) und Thallium-201-Diethydithiocarbamat (²⁰¹Tl-DDC). Beide Substanzen wurden daraufhin untersucht, ob sie sich für das eingesetzte bildgebende Verfahren bei Blutflussmessungen am Gehirn eignen.

Hierbei wurde festgestellt, dass beide Komplexverbindungen aufgrund ihrer Lipophilizität eine gute zerebrale Aufnahme zeigen, jedoch eine unterschiedlich starke Retention aufweisen. Dieser Unterschied in der Retention wurde dadurch erklärt, dass ²⁰¹Tl-DDC im Gehirn spontan zerfällt und ionisches ²⁰¹Tl gebildet wird, das die Blut-Hirn-Schranke nicht passieren kann. ^{99m}Tc-DDC hingegen besitzt eine wesentlich geringere Zerfallrate im Gehirn, weshalb die Verbindung dort in geringerem Maße zurückgehalten wird.

Trotz dieser Erkenntnisse wurde die Eignung von ²⁰¹Tl-DDC in dem beschrieben Verfahren unter anderem deshalb als gering eingeschätzt, da es sich aufgrund seiner Gamma-Emmission nicht optimal für SPECT-Verfahren eignet. Weiterhin wurde festgestellt, dass ²⁰¹Thallium aufgrund seiner Halbwertszeit von drei Tagen nachteilig ist.

Es ist bisher jedoch kein Ansatz bekannt, bei dem der Zerfall eines Metallchelatkomplexes in physiologischer Umgebung gezielt zur Diagnostik genutzt wurde. Auch ist nicht bekannt, dass Metallchelatkomplexe nach diesem Kriterium synthetisiert oder ausgewählt wurden.

Es wurde nicht das große diagnostische Potential erkannt, das dieser Zerfall lipophiler Schwermetall-Komplexe bei Passage der Blut-Hirn-Schranke und die damit verbundene Retention einen Weg für die Verwendung dieser Komplexe zur Untersuchung des Ionen-Metabolismus im ZNS eröffnet.

Sämtliche dokumentierte Bestrebungen liefen ausschließlich darauf hinaus, den Zerfall der eingesetzten Tracer in physiologischer Umgebung möglichst zu unterdrücken oder zumindest zu verzögern.

Und bis heute ist dieses Wissen nicht genutzt worden, um ein Verfahren zu entwickeln, dass eine direkte Messung der Veränderung des Kationen-Metabolismus, auch in vivo, ermöglicht.

In einer 2004 veröffentlichen Arbeit von Goldschmidt et al., Neuroimage 23(2):638-47, wurde die Verwendung von Thallium-Acetat in einem hochauflösenden nichtradioaktiven Verfahren. Dieses Verfahren wurde primär durchgeführt, um eine histochemische, hochauflösende Darstellung neuronaler Aktivität zu ermöglichen. Grundlage dafür ist, dass neuronale Aktivität und Kalium-(Thallium-) -Aufnahme bekannterweise eng gekoppelt sind und die Thalliumverbindung diente als Tracer für Kaliumionen. Grundlage des beschriebenen Verfahrens ist die so genannte Autometallographie, bei der es sich um ein Standardnachweisverfahren für Schwermetalle handelt. Erläutert wird die Anwendung dieses Verfahrens im Tiermodell, bei dem die Autometallographie als histochemisches Verfahren nach Gewebsentnahme durchgeführt wurde.

Nachteilig bei diesem Verfahren ist die Tatsache, dass sehr große Mengen Thalliumacetat eingesetzt werden mussten, um der Empfindlichkeit des Verfahrens zu entsprechen. Diese hohen Thallium-Dosen und die Tatsache, dass der Nachweis des Thallium histochemisch nach Gewebsentnahme erfolgt, machen den Einsatz des Verfahrens bei Menschen unmöglich. Auch für die Versuchstiere wäre die Dosis tödlich, jedoch werden bereits 15 Minuten nach der Gabe des Thalliumacetats die Versuchstiere getötet und die Gehirne zur histochemischen Untersuchung entnommen.

Abgesehen von der Unmöglichkeit mit diesem Verfahren am Menschen zu arbeiten, hat der Einsatz wasserlöslicher Thalliumsalze noch den Nachteil, daß die regionale Thalliumverteilung durch regionale Unterschiede in der Kalium-Leitfähigkeit der Blut-Hirnschranke mitbestimmt wird. Das limitiert den Einsatz insbesondere bei der Analyse krankhafter Veränderungen, bei denen auch die Blut-Hirnschranke verändert wird, und erschwert auch den Vergleich der zellulären Thallium-Aufnahme in verschiedenen Hirnregionen.

Aufgabe der vorliegenden Erfindung ist es deshalb, ein Diagnostikum bereitzustellen, das die Nutzung freier Schwermetallionen zur Diagnostik von Veränderungen des Kationen-Metabolismus im zentralen Nervensystem ermöglicht, ohne dass die Blut-Hirnschranke geöffnet wird.

Weiterhin ist es Aufgabe der vorliegenden Erfindung ein diagnostisches Verfahren bereitzustellen, bei dem Schwermetallionen als Tracer für verschiedene Kationen zur Untersuchung des Kationen-Metabolismus, auch in vitro, genutzt werden können.

Und darüber hinaus ist es eine Aufgabe der vorliegenden Erfindung, ein Verfahren zur Diagnostik von Veränderungen des Kationen-Metabolismus im zentralen Nervensystem bereitzustellen, das mit einem wesentlich geringeren apparativen Aufwand als bei der PET durchgeführt werden kann und somit beispielsweise auch im Praxisbetrieb von Nuklearmedizinern durchführbar ist.

Die Aufgabe wird gelöst durch die Bereitstellung eines Diagnostikums, enthaltend mindestens einen Komplex aus lipophilem Anion und Metallionen sowie Lösemittel, Hilfs- und/oder Trägerstoffe,

wobei das lipophile Anion Diethyldithiocarbamat (DDC),

und wobei die Metallionen ausgewählt sind aus Thallium-Isotopen,

Gelöst wird die Aufgabe der Erfindung durch ein Diagnostikum, enthaltend mindestens einen Komplex aus lipophilen Anionen und Metallionen, wobei das lipophile Anion Diethyldithiocarbamat (DDC) und wobei die Metallionen ausgewählt sind aus Thallium-Isotopen, zusammen mit Lösemitteln, Hilfs- und/oder Trägerstoffen.

Insbesondere bevorzugt ist ein Diagnostikum, das folgende Komplexverbindungen enthält:

Die Aufgabe der Erfindung wird gelöst durch die Verwendung eines Diagnostikums zur Untersuchung von Stoffwechselvorgängen im Gehirn und/oder zentralen Nervensystem (ZNS)

Bevorzugt ist ein Verfahren zur Untersuchung von Stoffwechselvorgängen im Gehirn und/oder ZNS, wobei man einem Säugetier ein erfindungsgemäßes Diagnostikum verabreicht und die Verteilung des in dem Diagnostikum enthaltenen Metallions im Gewebe misst.

Besonders bevorzugt ist, dass im erfindungsgemäßen Verfahren radioaktive Metallionen mittels nuklearmedizinischer Verfahren nachgewiesen werden und paramagnetische Metallionen mittels Kernresonanzverfahren nachgewiesen werden.

Weiterhin bevorzugt ist, dass im erfindungsgemäßen Verfahren der Nachweis der radioaktiven Metallionen mittels SPET- oder PET-Verfahren oder Gamma-Kamera erfolgt.

Insbesondere bevorzugt ist, dass im erfindungsgemäßen Verfahren der Nachweis der Nachweis der paramagnetischen Metallionen mittels Kernspintomographie-Verfahren erfolgt.

Darüber hinaus ist es bevorzugt, dass das erfindungsgemäße Verfahren als Langzeitmessung durchgeführt wird.

Gelöst wird die Aufgabe also durch die Bereitstellung des erfindungsgemäßen Diagnostikums und des erfindungsgemäßen Verfahrens, wodurch die direkte Untersuchung des Ionen-Metabolismus im Gehirn und/oder ZNS ermöglicht wird.

Es werden Diagnostika bereitgestellt, die ausgesuchte lipophile Schwermetallkomplexe enthalten, welche instabil sind und nach Passage durch die Blut-Hirn-Schranke in ihre ionischen Komponenten zerfallen. Durch diesen Zerfall werden Metallionen in den Extrazellularraum des Hirns entlassen.

So enthält in einem Ausführungsbeispiel der Erfindung das Diagnostikum einen Kalium-Tracer, nämlich einen lipophilen Thallium-Komplex, bevorzugt ²⁰¹Tl-DDC. Durch die Verwendung dieses Diagnostikums im erfindungsgemäßen Verfahren werden Veränderungen im Kalium-Stoffwechsel, wie sie z.B. bei Hirninfarkten, Hirntumoren oder degenerativen Erkrankungen auftreten, auch im ZNS erfasst.

Überdies ist es ein Vorteil, dass die erfindungsgemäßen Diagnostika die Durchführung des erfindungsgemäßen Verfahrens als Langzeitmessung ermöglichen. Insbesondere die Auswahl von radioaktiven Metallverbindungen mit vergleichsweise langen Halbwertszeiten, was bisher im Stand der Technik gegen deren Verwendung in diagnostischen Verfahren sprach, kann im erfindungsgemäßen Verfahren genutzt werden. Gerade bei der Untersuchung von Stoffwechselvorgängen neben der Momentaufnahme zur Feststellung des aktuellen Status auch die Beobachtung der Entwicklung eines Zustandes in Abhängigkeit von der Zeit von besonderem Interesse.

So wird es durch das erfindungsgemäße Verfahren als Langzeitmessung beispielsweise möglich, die Progression eines Hirninfarkts über die ersten Stunden nach Einweisung zu verfolgen. In diesem Fall bietet sich zum Beispiel durch die Verwendung eines lipophilen Thalliumkomplexes die einzigartige Möglichkeit der Untersuchung der Kinetik der Tl⁺-Aufnahme und/oder des Tl⁺-Verlustes, wodurch Daten von entscheidender Bedeutung für die Beurteilung einer Therapie erhalten werden können.

Und da mittels des erfindungsgemäßen Verfahrens eine Unterscheidung zwischen gesundem und geschädigtem Hirngewebe möglich wird, ist auch die Nutzung des Verfahrens als Referenz-Untersuchung zur Überprüfung des Hirntodes denkbar, wie sie vor fast jeder Organspende erforderlich ist.

Ein weiterer Vorteil des erfindungsgemäßen Verfahrens ist es, dass dieses insbesondere auch bei Komapatienten angewendet werden kann. Diese Patienten können nämlich regelmäßig nicht in einen Tomographen eingefahren werden.

Die Herstellung der im Diagnostikum enthaltenen KomplexVerbindungen erfolgt analog zu im Stand der Technik bekannten Verfahren.

Die Herstellung des erfindungsgemäßen Diagnostikums erfolgt in an sich bekannter Weise, indem die Komplexverbindung, gegebenenfalls in Kombination mit Lösemitteln, Hilfs- und/oder Trägerstoffen suspendiert oder löst. Dieser Zubereitung kann sich die Sterilisierung der Suspension oder Lösung anschließen.

Die Verabreichung des Diagnostikums erfolgt parenteral, wie es einem Durchschnittsfachmann bekannt ist.

Im erfindungsgemäßen Verfahren erfolgt der Nachweis der im Diagnostikum enthaltenen Metallionen in an sich bekannter Weise. Zum Nachweis eingesetzter radioaktiver Isotope werden die klassischen Verfahren SPET oder PET oder Gamma-Kamera genutzt. Zum Nachweis der paramagnetischen Metallionen können alle im Stand der Technik bekannten Kernspin-Verfahren eingesetzt werden.

Das folgende Beispiel erläutert die Erfindung:

### Beispiel

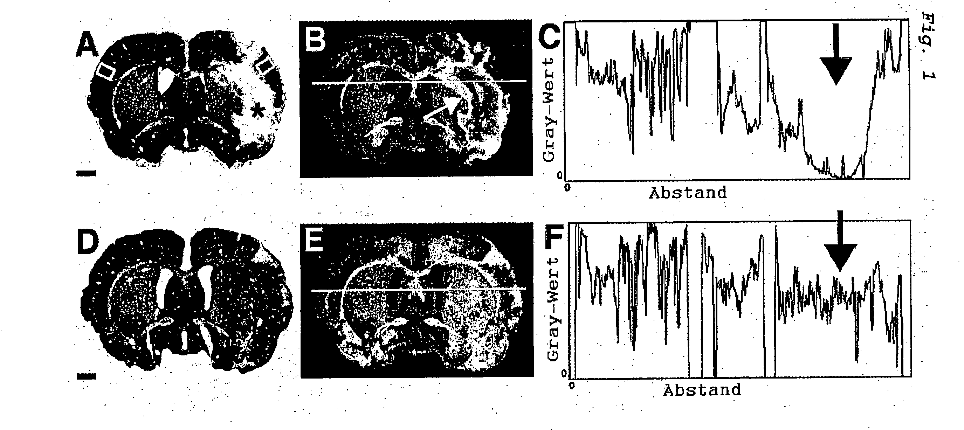

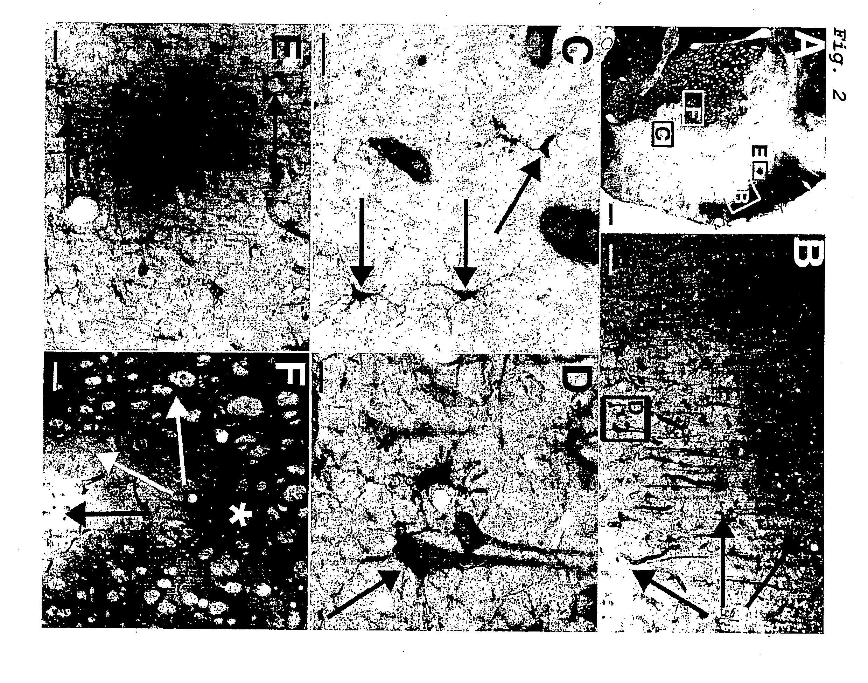

In den Figuren 1 und 2 ist die Verteilung von Thallium 201-Diethyldithiocarbamat (²⁰¹Tl-DDC) im Gehirn einer Ratte nach Injektion ²⁰¹Tl-DDC-Lösung gezeigt. Thallium ist ein Kalium-Tracer und in der Lage, Veränderungen im Kalium-Stoffwechsel, wie sie z.B. bei Hirninfarkten, Hirntumoren oder degenerativen Erkrankungen auftreten, zu erfassen.

Die Fig. 1 ist eine Übersicht über die Thalliumverteilung nach Tl-DDC Injektion im Gehirn der Ratte 15 Minuten (hyperakutes Stadium, A, B, C) und 7 Tage (D, E, F) nach fokaler cerebraler Ischämie. Gezeigt werden Frontalschnitte in der Ebene der anterioren Kommissur (A, D) und farbcodierte Bilder der gleichen Schnitte (B, E). Die Variation der optischen Dichte entlang der weißen Linien in B und E ist in C bzw. E dargestellt.
Im hyperakuten Stadium ist ein Infarktkern zu sehen (Stern in A und B, Pfeil in C), der eine deutlich verminderte Färbeintensität aufweist und mit herkömmlichen Techniken zu diesem frühen Zeitpunkt so nicht nachgewiesen werden kann. Dieses Gebiet ist umgeben von einer Zone, in der die Farbintensität höher ist, aber immer noch deutlich niedriger als in der nicht geschädigten kontralateralen Seite (Falschfarbendarstellung in Grün, weißer Pfeil in B).
Im späten Zeitpunkt (D, E, F) sind die Unterschiede ipsi- und kontralateral zum verschlossenen Gefäß weniger ausgeprägt (Balken in A und D 1 mm).

Die Fig. 2 zeigt ein hochaufgelöstes Bild der Thallium Verteilung im hyperakuten Stadium der cerebralen Ischämie. Die Rechtecke in der Übersichtsaufnahme (A) markieren die Position der in B, C, E und F gezeigten Details. Sowohl im cerebralen Kortex (B) als auch im Caudatoputamen (F) können Gebiete unterschiedlicher Färbeintensität unterschieden werden (Pfeile in B und F). Die Thalliumaufnahme erfolgt im Wesentlichen durch die Natrium-Kaliumpumpe und über sie regulierte Transportsysteme und ist somit energieabhängig. Zellen, die das benötigte E-nergieäquivalent ATP nicht mehr produzieren können färben sich nicht und Zellen mit verminderter Pumpleistung nur schwach (z.B. Neuron in D, Stern). Daher kann Thallium in den Neuronen des Infarktkerns nicht nachgewiesen werden (C), wohingegen die metabolisch hochaktiven Astrozyten (Pfeile in C) intensiv gefärbt sind. Diese metabolischen Unterschiede auf zellulärem Niveau sind mit den bisherigen Methoden, insbesondere auch zu so frühen Zeitpunkten nach Ischämie, nicht nachweisbar. Die Astrozyten weisen auch in der direkt an das Kerngebiet angrenzenden Zone einen erhöhten Metabolismus und damit eine starke Färbung auf (Pfeil in D, Detail aus B). Mit der Technik können metabolische Unterschiede selbst räumlich eng beieinanderliegende Zellen Astrozyt und Neuron in D, Pfeil) dargestellt werden. Sowohl im cerebralen Kortex (Pfeil in E) als auch im Caudatoputamen (Stern in F) können in den Randzonen des Infarktgebiets auch Neurone und Neuropil mit hoher metabolischer Aktivität nachgewiesen werden. Balken in A 500 µm, in B 50 µm, in C 25 µm, in D 10 µm, in E 25 µm und in F 100 µm.

## Patentansprüche

1. Verwendung eines Diagnostikums enthaltend mindestens einen Komplex aus einem lipophilen Anion und einem Metallion sowie Lösemittel, Hilfs- und/oder Trägerstoffe, wobei das lipophile Anion Diethyldithiocarbamat (DDC) ist und das Metallion ausgewählt ist aus Thallium-Isotopen,
zur Untersuchung von Stoffwechselvorgängen im Gehirn und/oder zentralen Nervensystem (ZNS).

2. Verwendung gemäß Anspruch 1, **dadurch gekennzeichnet, dass** man einem Säugetier das genannte Diagnostikum verabreicht und die Verteilung des in dem Diagnostikum enthaltenen Metallions im Gewebe misst.

3. Verwendung gemäß Anspruch 2, **dadurch gekennzeichnet, dass** man radioaktive Metallionen mittels nuklearmedizinischer Verfahren nachweist und paramagnetische Metallionen mittels Kernresonanzverfahren nachweist.

4. Verwendung gemäß Anspruch 3, **dadurch gekennzeichnet, dass** der Nachweis der radioaktiven Metallionen mittels SPET- oder PET-Verfahren oder Gamma-Kamera erfolgt.

5. Verwendung gemäß Anspruch 3, **dadurch gekennzeichnet, dass** der Nachweis der paramagnetischen Metallionen mittels Kernspintomographie-Verfahren erfolgt.

6. Verwendung gemäß einem der Ansprüche 1 bis 5, **dadurch gekennzeichnet, dass** man die genannte Untersuchung als Langzeitmessung durchführt.

## Claims

1. Use of a diagnostic substance, containing at least one complex of lipophilic anion and one metal ion as well as solvents, adjuvants and/or vehicles, wherein the liophilic anion is diethyldithiocarbamate (DDC) and the metal ion is selected from thallium isotopes,
for investigating metabolic processes in the brain and/or central nervous system (CNS).

2. Use according to claim 1, **characterized in that** the diagnostic substance is administered to a mammal and the distribution of the metal ions contained in the diagnostic substance in tissue is measured.

3. Use according to claim 2, **characterized in that**, radioactive metal ions are detected by means of nuclear medicine methods and paramagnetic metal ions are detected by means of nuclear resonance methods.

4. Use according to claim 3, **characterized in that** the detection of the radioactive metal ions is performed by means of SPET or PET methods or a gamma camera.

5. Use according to claim 3, **characterized in that** the detection of the paramagnetic metal ions is performed by means of nuclear spin tomography methods.

6. Use according to one of claims 1 to 5, **characterized in that** said investigation is conducted as long-term measurement.

## Revendications

1. Utilisation d'un agent diagnostique comprenant au moins un complexe formé à partir d'un anion lipophile et d'un ion métallique, ainsi que des solvants, des adjuvants et/ou des substances de support, l'anion lipophile étant le dithiocarbamate de diéthyle (DDC) et l'ion métallique étant choisi parmi des isotopes du thallium, pour l'examen de processus métaboliques dans le cerveau et/ou dans le système nerveux central (SNC).

2. Utilisation selon la revendication 1, **caractérisée en ce qu'**on administre à un mammifère l'agent diagnostique mentionné et on mesure la distribution tissulaire de l'ion métallique contenu dans l'argent diagnostique.

3. Utilisation selon la revendication 2, **caractérisée en ce qu'**on décèle des ions métalliques radioactifs au moyen de procédés de la médecine nucléaire et on décèle des ions métalliques paramagnétiques au moyen de procédés de résonance nucléaire.

4. Utilisation selon la revendication 3, **caractérisée en ce que** le décèlement des ions métalliques radioactifs a lieu au moyen du procédé SPET ou PET ou à l'aide d'une gamma-caméra.

5. Utilisation selon la revendication 3, **caractérisée en ce que** le décèlement des ions métalliques paramagnétique a lieu au moyen de procédés de résonance magnétique nucléaire.

6. Utilisation selon l'une quelconque des revendications 1 à 5, **caractérisée en ce qu'**on effectue l'examen mentionné sous la forme d'une mesure à long terme.
